# FASCICULE DE BREVET EUROPEEN

(11) **EP 2 347 296 B1**
(45) Date de publication et mention de la délivrance du brevet: **06.01.2016**
(21) Numéro de dépôt: 09771391.1
(22) Date de dépôt: 28.10.2009
(51) Int. Cl.: G02B 21/36, G02B 21/00, G01N 1/30, G01N 33/569

(54) **Procédure de préparation d'une image d'analyse virtuelle traitée**
Verfahren zum Erzeugen eines verarbeiteten Bildes zur virtuellen Analyse
Procedure for preparing a processed virtual analysis image

(30) Priorité: 31.10.2008 FR 0857438
(43) Date de publication de la demande: 27.07.2011
(73) Titulaire: NOVACYT, 78140 Velizy Villacoublay (FR)
(72) Inventeur: PELTIER, Eric, F-92140 Clamart (FR)
(74) Mandataire: Blot, Philippe Robert Emile
(86) Numéro de dépôt international: PCT/FR2009/052086
(87) Numéro de publication internationale: WO 2010/049651

(56) Documents cités:
- EP-A- 1 324 097
- EP-A- 1 830 216
- WO-A-01/84209
- WO-A-98/39728
- US-A- 4 760 385

## Description

La présente invention concerne un procédé de préparation d'une plaque d'analyse virtuelle d'un prélèvement cytologique ou histologique disposé sur une plaque d'analyse en vue de permettre l'analyse cellulaire dudit prélèvement, tel que défini dans la revendication 1.

L'invention s'applique particulièrement à des procédés d'analyse cytologique ou histologique.

L'invention concerne également un procédé de préparation d'une plaque d'analyse virtuelle d'un prélèvement en vue de permettre son analyse cellulaire, notamment selon un procédé d'analyse tel que décrit ci-dessus.

L'analyse de prélèvements est par exemple utilisée pour le diagnostic de pathologies, par exemple à partir de cellules prélevées par frottis (cervicaux, vaginaux ou autres), par ponction d'organes (sein, thyroïde, ganglion ou autres) ou encore par recueil (urine, lavage broncho-alvéolaire ou autres), pour détecter tout type de pathologie et plus particulièrement des états pré cancéreux ou cancéreux.

Les prélèvements sont examinés par des observateurs spécialisés et entraînés pour détecter les cellules susceptibles d'être pathologiques dans un prélèvement disposé sur une plaque ou lame d'analyse. Afin de permettre la détection de cellules potentiellement pathologiques, le prélèvement subit un traitement, tel qu'une coloration permettant de mettre en évidence entre autres, les caractéristiques du noyau et du cytoplasme des cellules afin d'aider au repérage et au diagnostic des cellules pathologiques. Lorsque le prélèvement est observé, les cellules potentiellement pathologiques montrent alors des différences d'affinités tinctoriales, de taille et de forme, tant au niveau du noyau que du cytoplasme, par rapport aux cellules normales.

L'analyse peut se faire de façon manuelle, sans assistance particulière. Dans ce cas, le médecin ou le technicien spécialisé fait défiler des plaques de prélèvement sous un microscope et observe chacune d'entre elles en vue de détecter les anomalies morphologiques indiquant des cellules pathologiques pouvant correspondre à un état pré cancéreux ou cancéreux par exemple. Une telle méthode d'analyse est fastidieuse et consomme énormément de temps. En outre, elle n'offre pas de résultats satisfaisants avec surtout un nombre de « faux négatifs » estimés à environ 30%, c'est-à-dire de prélèvements considérés comme normaux alors qu'il existe une pathologie chez le patient, notamment pré cancéreuse ou cancéreuse avec les risques d'évolution ultérieure du cancer chez un patient rassuré à tort.

Pour améliorer les résultats des analyses, il a été proposé d'améliorer l'échantillonnage, c'est-à-dire le nombre de cellules, leur fixation, leur coloration et leur étalement sur la plaque d'analyse, mais aussi d'aider dans son analyse le médecin ou le technicien spécialisé par exemple grâce à des moyens informatiques d'analyse, tels que des logiciels de traitement d'images et autre.

A cet effet, une caméra ou un appareil de prise de vues est utilisé pour acquérir des images des différentes zones du prélèvement disposé sur la plaque d'analyse et communiquer les données de ces images à un système informatique qui travaille alors sur une plaque d'analyse « virtuelle ».

Ce système informatique permet un traitement du signal, un pré traitement des images et une analyse comparative des images avec éventuellement des bases de données nouvellement créées ou existantes afin d'accélérer le processus d'analyse et ainsi permettre l'analyse d'un plus grand nombre de prélèvements et afin d'assister le médecin ou le technicien spécialisé. Les images d'un prélèvement sont par exemple examinées automatiquement et si certaines zones présentant une anomalie sont relevées, les images correspondantes sont communiquées à un médecin ou le technicien spécialisé qui peut alors déterminer si oui ou non ces zones montrent des cellules pathologiques. Le médecin ou le technicien spécialisé n'observe donc plus que des zones anormales sans analyser les zones considérées comme normales par le système informatique. Un tel procédé permet effectivement d'accélérer l'analyse et de fiabiliser le diagnostic. La demande Européenne, EP 1 324 097 A divulgue un système et un procédé de préparation d'une plaque d'analyse histologique, le procédé comprenant des étapes de traitement d'un échantillon prélevé d'un organe avec un colorant, d'acquisition d'images sur la plaque d'analyse de sorte à obtenir une pluralité d'images représentant chacune une zone de la plaque d'analyse, lesdites images mises côté-à-côté formant une image de l'intégralité de sorte à créer une plaque d'analyse virtuelle, et des étapes de traitement de l'image virtuelle par des logiciels.

Cependant le médecin ou le technicien spécialisé n'a alors plus l'occasion d'observer des prélèvements normaux ou présentant des modifications morphologiques mineures, ce qui est préjudiciable à son appréciation des prélèvements et surtout pour sa courbe d'apprentissage voire la conservation de son acuité diagnostique. En effet, l'analyse de prélèvements repose sur la formation et la pratique du médecin ou du technicien spécialisé à examiner des prélèvements et à comparer des zones normales et des zones présentant des anomalies. Le fait de supprimer cette pratique par une analyse assistée par ordinateur peut mener les médecins ou techniciens spécialisés à perdre leurs compétences et ainsi entraîner des erreurs d'analyse.

En outre, l'opération de numérisation peut prendre un temps considérable du fait des traitements appliqués sur la lame avant la numérisation.

L'invention vise à pallier les inconvénients mentionnés ci-dessus en proposant un procédé d'analyse qui, tout en étant assisté afin de permettre un gain de temps notable, notamment au cours de l'opération de numérisation, permet également aux médecins ou techniciens spécialisés de s'entraîner à observer des prélèvements autant normaux qu'anormaux.

A cet effet, l'invention concerne un procédé du type précité comprenant en outre l'étape suivante :
- effectuer sur la plaque d'analyse virtuelle un traitement des images acquises de sorte à obtenir une restitution virtuelle des couleurs et de l'intensité des couleurs du cytoplasme et/ou du noyau, lesdites couleurs et ladite intensité pouvant être modifiées selon les préférences de la personne en charge de l'analyse.

Un tel procédé permet de réaliser de façon particulièrement simple une image d'un prélèvement permettant de détecter des cellules éventuellement pathologiques et d'acquérir un grand nombre d'informations sur le prélèvement, comme cela sera décrit ultérieurement.

Selon d'autre caractéristiques du procédé de préparation :
- le procédé comprend en outre une étape de superposition des données des images acquises lors de l'acquisition des images et celles des images acquises modifiées lors du traitement des images acquises,
- le traitement du prélèvement est une étape de coloration nucléaire ou une coloration de cytologie, ledit traitement étant agencé pour rendre le cytoplasme presque transparent et accentuer le contraste entre le noyau et/ou l'ARN cytoplasmique des cellules et le cytoplasme,
- le traitement des images acquises correspond à la recoloration virtuelle de type Papanicolaou, Schorr, May Grunwald Giemsa ou Giemsa de ces images acquises,
- le niveau de recoloration virtuelle des images acquises est réglable,
- le procédé comprend une étape d'affichage de la plaque d'analyse virtuelle,
- l'affichage de la plaque d'analyse est accompagné de l'affichage d'informations sur la zone de prélèvement affichée et/ou sur l'intégralité du prélèvement et/ou sur le patient sur lequel le prélèvement a été effectué et/ou sur le résultat d'examens complémentaires réalisés sur le prélèvement,
- le procédé comprend une étape d'agrandissement de la plaque d'analyse virtuelle afin de permettre le visionnage d'un détail de ladite plaque,
- l'agrandissement est réglable.

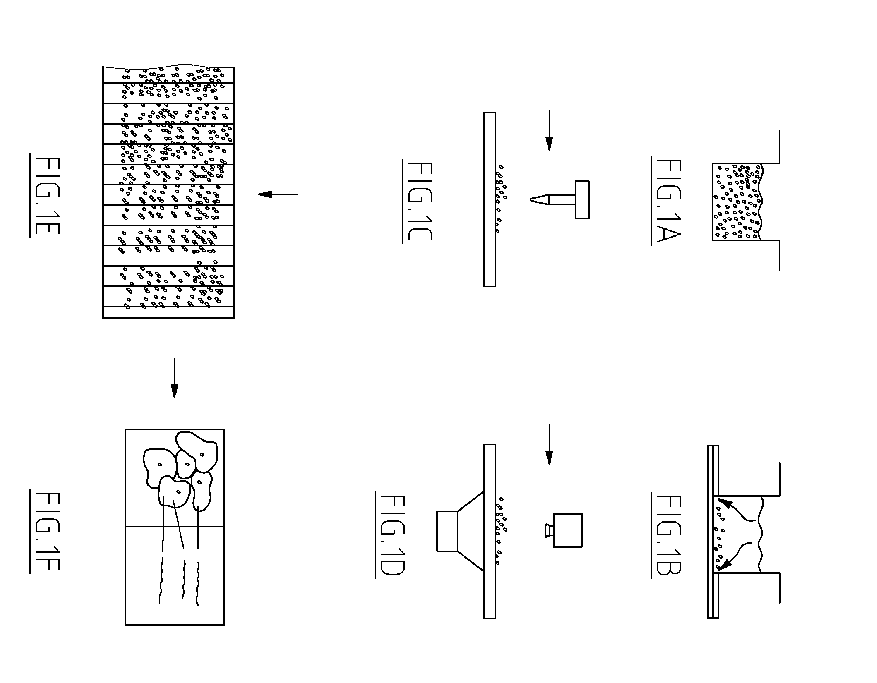

D'autres aspects de l'invention apparaîtront à la lecture de la description qui suit, donnée à titre d'exemple et faite en référence au dessin annexé qui est une représentation schématique des différentes étapes du procédé de préparation d'une plaque d'analyse virtuelle selon l'invention.

En référence à la figure, on décrit un procédé de préparation d'une plaque d'analyse virtuelle en vue de son analyse cellulaire assistée par un système informatique. Par plaque virtuelle, on entend un ensemble d'informations et de données numériques regroupées concernant un prélèvement.

Le prélèvement est par exemple obtenu par frottis (cervicaux, vaginaux ou autres), par ponction d'organes (sein, thyroïde, ganglion ou autres) ou encore par recueil (urine, lavage broncho-alvéolaire ou autres). Au cours d'une première étape A, le prélèvement est mis en suspension, par exemple dans un tube ou un flacon de prélèvement.

Au cours d'une étape B, le prélèvement est disposé sur une plaque d'analyse. De façon connue, le dépôt des cellules sur la plaque se fait par exemple par décantation. Le prélèvement est versé dans une chambre de décantation dont le fond est ouvert sur la plaque d'analyse. Des moyens d'absorption permettent l'absorption de la solution au fur et à mesure que les cellules se déposent sur la plaque d'analyse. Un tel procédé de dépôt est connu et ne sera pas décrit en détail ici.

Au cours d'une étape C, le prélèvement subit un premier traitement visant à marquer/colorer les noyaux, l'ADN et/ou l'ARN des cellules en accentuant très nettement le contraste par rapport aux cytoplasmes de ces mêmes cellules. Un tel marquage permet de correctement segmenter les noyaux pour en faire l'étude morphologique et pour faire une quantification de l'ADN (pour une analyse de la ploïdie par exemple) et/ou de l'ARN afin de repérer les cellules potentiellement pathologiques.

Ce marquage est par exemple réalisé de façon automatique au moyen d'un automate muni de moyens de pipetage utilisés à la fois pour la mise en solution du prélèvement et pour le dépôt de la suspension cellulaire sur une plaque d'analyse.

L'étape de décantation de la suspension cellulaire peut être réalisée avant, après ou entre les étapes de marquage et/ou de coloration décrites ci-dessus.

D'autres colorations utilisées dans l'état de la technique pourraient être utilisées, mais elles présentent des inconvénients. Ainsi, on pourrait envisager une coloration stoechiométrique, qui offre une coloration des cellules proportionnelle à la quantité d'ADN, ce qui permet sa quantification, et donc de repérer et d'analyser les cellules pathologiques dans le cadre de la ploïdie. Cependant, cette coloration particulière, lorsqu'il s'agit d'une coloration de Feulgen par exemple, est incompatible « physiquement » avec la coloration de Papanicolaou et nécessite donc de refaire un étalement cellulaire sur lame pour l'analyse du médecin ou du technicien spécialisé. Certains industriels ont essayé d'associer une coloration stoechiométrique avec la coloration de Papanicolaou et ont donc utilisé une coloration renfermant une thionine et nécessitant une fixation au méthanol qui est toxique, et surtout qui modifie la coloration de Papanicolaou dans son interprétation, notamment avec des noyaux dont la chromatine apparaît trop « noire » pour une analyse fine de la composition desdits noyaux, et donc entraîne une difficulté d'analyse pour le diagnostic d'états pré cancéreux ou cancéreux.

Au cours d'une étape D, la plaque d'analyse comprenant le prélèvement, coloré par un colorant nucléaire connu ou par une coloration cytologique connue mais modifiée pour rendre le cytoplasme presque transparent, est soumis à un éclairement en lumière blanche afin d'acquérir des images du prélèvement au moyen du dispositif d'acquisition d'images. L'acquisition d'images en lumière blanche permet d'obtenir des images du prélèvement coloré par la coloration nucléaire ou la coloration cytologique modifiée conservant des cytoplasmes presque transparents afin d'augmenter le contraste avec les noyaux.

L'appareil d'acquisition d'images permet de « scanner » le prélèvement avec une résolution très fine et d'obtenir à partir d'une plaque, des images en lumière blanche. La plaque d'analyse est scannée ligne par ligne ou champs par champs comme le propose de nombreux industriels. Ainsi, chaque image acquise représente une bande de faible largeur de la plaque d'analyse ou champs de vue. Les images mises côte à côte permettent d'obtenir une image de l'intégralité de la plaque de prélèvement et donc de l'intégralité du prélèvement afin de former une plaque d'analyse virtuelle, comme représenté à l'étape E de la figure. Ainsi, l'image, obtenue avec un seul et même appareil et de façon très simple, est une représentation fidèle du prélèvement associant un grand nombre d'informations en lumière blanche.

Les données numériques obtenues avec l'appareil en lumière blanche subissent un traitement informatique afin d'obtenir une plaque d'analyse virtuelle modifiée formée de l'image ayant subit une recoloration virtuelle de type Papanicolaou, Schorr, May Grunwald Giemsa ou Giemsa visant à colorer les cellules tel que classiquement décrit et connu pour une analyse cytologique par l'homme de l'art. Selon un mode de traitement informatique, la recoloration virtuelle de Papanicolaou, obtenue telle que connue depuis longtemps et dont la sémiologie, largement décrite dans la littérature, permet la reconnaissance éventuelle d'anomalies cytoplasmiques et nucléaires correspondant par exemple à la présence de cellules pré cancéreuses ou cancéreuses. Cette recoloration permet également la reconnaissance des différents types de cellules et de leur nombre afin de déterminer la qualité représentative du prélèvement et, par exemple de définir si le prélèvement est représentatif ou non. Cette recoloration virtuelle permet l'analyse de la plaque virtuelle par un procédé d'analyse cellulaire décrit ci-dessous.

L'analyse cellulaire se fait par un examen des images du prélèvement par le médecin ou le technicien spécialisé chargé de détecter les cellules pathologiques, pour proposer un diagnostic qui déclenchera éventuellement des examens plus approfondis voire un traitement. Pour des raisons de sécurité, la présence du médecin ou du technicien spécialisé est obligatoire de sorte que la détection de cellules éventuellement pathologiques ne peut pas être entièrement automatisée.

Au cours d'une étape F, les images de la plaque d'analyse virtuelle modifiée formée de l'image ayant subit une recoloration virtuelle sont projetées sur un moyen d'affichage tel qu'un écran. La restitution virtuelle des couleurs et de leur intensité tant pour le cytoplasme que pour le noyau, peut être réglée de façon à correspondre au plus près des habitudes de chaque lecteur, c'est-à-dire le médecin ou le technicien spécialisé, en terme d'intensité et de qualité de coloration.

Puis, ces images défilent sous les yeux du médecin ou du technicien spécialisé pour examen. Le défilement des images est organisé par le système informatique et se fait de manière automatique. Chaque image est affichée pendant un temps prédéterminé calculé pour permettre au médecin ou au technicien spécialisé d'observer l'intégralité de chaque image projetée et de détecter une éventuelle anomalie dans une image. Le temps d'affichage de chaque image peut être réglé par le médecin ou le technicien spécialisé en fonction de ses compétences ou en fonction d'autres informations. Les informations sur le patient peuvent être associées à la plaque d'analyse virtuelle en entrant ces informations dans une base de données et en les associant à l'image du prélèvement correspondant au patient sur lequel ce prélèvement a été effectué.

L'affichage des images peut être accompagné de l'affichage d'informations sur la zone de prélèvement affichée et/ou sur l'intégralité du prélèvement et/ou sur le patient sur lequel le prélèvement a été effectué et/ou sur le résultat d'examens complémentaires réalisés sur le prélèvement, notamment d'examens de biologie moléculaire.

Les images qui défilent sont donc celles de la plaque d'analyse virtuelle modifiée, formée de l'image ayant subit une recoloration virtuelle de type Papanicolaou, Schorr, May Gruwald Giemsa ou Giemsa. Les images sous cette coloration permettent de détecter la présence éventuelle d'anomalies correspondant par exemple à la présence de cellules pré cancéreuses ou cancéreuses et dont la sémiologie connue depuis longtemps est largement décrite dans la littérature. En outre, comme indiqué plus haut, la coloration de Papanicolaou permet de vérifier si le prélèvement répond bien aux critères de Bethesda, dans le cadre du frottis du col de l'utérus par exemple, et est donc un prélèvement valable ou non. Les critères de Bethesda peuvent être vérifiés de façon automatique par le système informatique au moyen d'un logiciel d'analyse d'images. Ce logiciel peut par exemple effectué un comptage des cellules saines ou pathologiques. Le comptage de toutes les cellules sur l'image permet de vérifier si au moins 5000 cellules ont bien été prélevées et si certains types de cellules témoin de la bonne qualité du prélèvement, telles que des cellules endocervicales ou de la jonction, ont été prélevés. Le logiciel peut également acquérir d'autres informations sur l'étalement cellulaire. Ces informations sont rattachées à l'image par le système informatique pour compléter la plaque d'analyse virtuelle.

L'image affichée sous recoloration virtuelle, et pour laquelle le défilement a été interrompu, est associée à l'image de la même zone sans recoloration virtuelle. Cet affichage permet au médecin ou au technicien spécialisé d'affiner son analyse des cellules affichées et de confirmer ou non si certaines sont éventuellement pathologiques. En outre, avec l'affichage de l'image sans recoloration virtuelle, d'autres informations peuvent être affichées simultanément, telles que des données quantitatives, des spectres ou des informations sur le patient sur lequel le prélèvement a été effectué, etc. Cette analyse plus fine couplée à l'arrêt automatisé du défilement permet de diminuer le nombre de faux négatifs. De plus, dans le cadre du frottis du col de l'utérus par exemple, le contrôle du diagnostic par le médecin ou le technicien spécialisé des zones sélectionnées par le système permet de conserver le niveau de spécificité du diagnostic cytologique qui est proche de 95% dans ce cas. De ce fait, les critères de sensibilité et de spécificité du frottis de dépistage deviennent plus proches et élevés.

Au cours de cette étape, le médecin ou le technicien spécialisé peut librement effectuer un grossissement de zones particulières de l'image affichée, aussi bien sur l'image avec recoloration virtuelle que sur l'image sans recoloration virtuelle. Le médecin ou le technicien spécialisé peut également basculer de l'image avec recoloration virtuelle à l'image sans recoloration virtuelle comme bon lui semble.

Le procédé décrit ci-dessus permet une analyse rapide et efficace de prélèvements, diminuant le risque de « faux négatifs ».

En outre, l'expérience des médecins ou techniciens spécialisés en terme de qualité diagnostique et surtout de spécificité, est préservé du fait de la reconnaissance des couleurs et de leur intensité liée aux images virtuelles des préparations cytologiques et/ou histologiques traitées et affichées.

Le procédé permet également d'adapter la plaque d'analyse aux préférences du médecin ou technicien spécialisé chargé de l'analyse. En effet, le médecin ou technicien spécialisé peut choisir l'intensité et la coloration de la plaque d'analyse virtuelle à sa guise.

## Revendications

1. Procédé de préparation d'une plaque d'analyse virtuelle d'un prélèvement cytologique ou histologique disposé sur une plaque d'analyse en vue de permettre l'analyse cellulaire dudit prélèvement, comprenant les étapes suivantes :
- réaliser un traitement de marquage du prélèvement, ledit traitement étant réalisé pour permettre de différencier les cellules pathologiques des cellules saines du prélèvement,
- effectuer une acquisition d'images du prélèvement disposé sur la plaque d'analyse de sorte à obtenir une pluralité d'images représentant chacune une zone de la plaque d'analyse, lesdites images mises côte à côte formant une image de l'intégralité du prélèvement de sorte à créer une plaque d'analyse virtuelle, **caractérisé en ce qu'**il comprend en outre l'étape suivante :
- effectuer sur la plaque d'analyse virtuelle un traitement de recoloration virtuelle de type Papanicolaou, Schorr, May Grunwald Giemsa ou Giemsa de ces images acquises.

2. Procédé de préparation selon la revendication 1, **caractérisé en ce qu'**il comprend en outre une étape de superposition des données des images acquises lors de l'acquisition des images et celles des images acquises modifiées lors du traitement des images acquises.

3. Procédé de préparation selon la revendication 1 ou 2, **caractérisé en ce que** le traitement du prélèvement est une étape de coloration nucléaire ou une coloration de cytologie, ledit traitement étant agencé pour rendre le cytoplasme presque transparent et accentuer le contraste entre le noyau et/ou l'ARN cytoplasmique des cellules et le cytoplasme.

4. Procédé de préparation selon l'une quelconque des revendications 1 à 4, **caractérisé en ce que** le niveau de recoloration virtuelle des images acquises est réglable.

5. Procédé de préparation selon l'une quelconque des revendications 1 à 5, **caractérisé en ce qu'**il comprend une étape d'affichage de la plaque d'analyse virtuelle.

6. Procédé de préparation selon l'une quelconque des revendications 1 à 7, **caractérisé en ce qu'**il comprend une étape d'agrandissement de la plaque d'analyse virtuelle afin de permettre le visionnage d'un détail de ladite plaque.

7. Procédé de préparation selon la revendication 8, **caractérisé en ce que** l'agrandissement est réglable.

## Patentansprüche

1. Erzeugungsverfahren einer virtuellen Analyseplatte einer auf einer Analyseplatte zwecks Durchführung einer Zellanalyse der Probe aufgebrachten zytologischen oder histologischen Probe, das die folgenden Schritte umfasst:
- Durchführen einer Markierungsbehandlung der Probe, wobei die Behandlung durchgeführt wird, um die Unterscheidung der pathologischen Zellen von den gesunden Zellen der Probe zu erlauben,
- Durchführen einer Bildaufnahme der auf der Analyseplatte aufgebrachten Probe, um eine Vielzahl von Bildern zu erhalten, die jeweils eine Zone der Analyseplatte darstellen, wobei die nebeneinandergelegten Bilder ein Bild der gesamten Probe ergeben, um eine virtuelle Analyseplatte zu bilden,
**dadurch gekennzeichnet, dass** es ferner den folgenden Schritt umfasst:
- Durchführen einer virtuellen Anfärbehandlung vom Typ Papanicolaou, Schorr, May Grundwald Giemsa oder Giemsa dieser aufgenommenen Bilder auf der virtuellen Analyseplatte.

2. Erzeugungsverfahren nach Anspruch 1, **dadurch gekennzeichnet, dass** es ferner einen Überlagerungsschritt der Daten der bei der Bildaufnahme aufgenommen Bilder und der Daten der bei der Verarbeitung der aufgenommenen Bilder geänderten aufgenommenen Bilder umfasst.

3. Erzeugungsverfahren nach Anspruch 1 oder 2, **dadurch gekennzeichnet, dass** die Behandlung der Probe ein Anfärben der Zellkerne oder ein zytologisches Anfärben ist, wobei die Behandlung ausgebildet ist, um das Zytoplasma fast durchsichtig zu machen und den Kontrast zwischen dem Zellkern und/oder der zytoplasmischen RNA der Zellen und des Zytoplasmas zu akzentuieren.

4. Erzeugungsverfahren nach einem der Ansprüche 1 bis 4, **dadurch gekennzeichnet, dass** die Stärke der virtuellen Anfärbung der aufgenommenen Bilder einstellbar ist.

5. Erzeugungsverfahren nach einem der Ansprüche 1 bis 5, **dadurch gekennzeichnet, dass** es einen Anzeigeschritt der virtuellen Analyseplatte umfasst.

6. Erzeugungsverfahren nach einem der Ansprüche 1 bis 7, **dadurch gekennzeichnet, dass** es einen Vergrößerungsschritt der virtuellen Analyseplatte umfasst, um die Betrachtung eines Details der Platte zu erlauben.

7. Erzeugungsverfahren nach Anspruch 8, **dadurch gekennzeichnet, dass** die Vergrößerung einstellbar ist.

## Claims

1. A method for preparing a virtual analysis plate of a cytological or histological specimen disposed on an analysis plate with view to allowing cell analysis of said specimen, comprising the following steps:
- carrying out a processing for staining the specimen, said processing being carried out so as to make it possible to differentiate the pathological cells from the healthy cells of the specimen,
- performing an acquisition of images of the specimen disposed on the analysis plate, so as to obtain a plurality of images each representing a zone of the analysis plate, said images placed side by side forming an image of the whole of the specimen so as to create a virtual analysis plate,
**characterized in that** it further comprises the following step:
- performing on the virtual analysis plate a treatment for virtual re-coloration of the Papanicolaou, Schorr, May Grunwald Giemsa or Giemsa type of these acquired images.

2. The preparation method according to claim 1, **characterized in that** it further comprises a step for superimposing the data of the images acquired during acquisition of the images and of those of the acquired images modified during the processing of the acquired images.

3. The preparation method according to claim 1 or 2, **characterized in that** the treatment of the specimen is a nuclear or cytological coloration step, said treatment being laid out so as to make the cytoplasm almost transparent and to enhance the contrast between the nucleus and/or the cytoplasm RNA of the cells and the cytoplasm.

4. The preparation method according to any of claims 1 to 3, **characterized in that** the virtual re-coloration level of the acquired images is adjustable.

5. The preparation method according to any of claims 1 to 4, **characterized in that** it comprises a step for displaying the virtual analysis plate.

6. The preparation method according to any of claims 1 to 5, **characterized in that** it comprises a step for enlarging the virtual analysis plate in order to allow viewing of a detail of said plate.

7. The preparation method according to claim 6, **characterized in that** the enlargement is adjustable.
